# EUROPEAN PATENT SPECIFICATION

(11) **EP 2 157 973 B1**
(45) Date of publication and mention of the grant of the patent: **15.07.2015**
(21) Application number: 08764779.8
(22) Date of filing: 21.05.2008
(51) Int. Cl.: A61K 31/445, A61K 31/4709, A61P 25/28

(54) **A MEDICAMENT COMPRISING A CARBOSTYRIL DERIVATIVE AND DONEPEZIL FOR TREATING ALZHEIMER'S DISEASE**
MEDIKAMENT MIT EINEM CARBOSTYRIL-DERIVAT UND DONEPEZIL ZUR BEHANDLUNG DER ALZHEIMER-KRANKHEIT
MÉDICAMENT COMPRENANT UN DÉRIVÉ DE CARBOSTYRILE ET DE DONÉPÉZIL DESTINÉ À TRAITER LA MALADIE D'ALZHEIMER

(30) Priority: 22.05.2007 JP 2007135367
(43) Date of publication of application: 03.03.2010
(60) Divisional application: 15164200.6
(73) Proprietor: OTSUKA PHARMACEUTICAL CO., LTD., Tokyo 101-8535 (JP); Juntendo University, Tokyo 113-8421 (JP)
(72) Inventor: ARAI, Heii, Tokyo 113-8421 (JP)
(74) Representative: Von Kreisler Selting Werner - Partnerschaft von Patentanwälten und Rechtsanwälten mbB
(86) International application number: PCT/JP2008/059763
(87) International publication number: WO 2008/143361

(56) References cited:
- WO-A-2004/075897
- US-A1- 2004 229 913
- LEE ET AL: "Concurrent administration of cilostazol with donepezil effectively improves cognitive dysfunction with increased neuroprotection after chronic cerebral hypoperfusion in rats" BRAIN RESEARCH, ELSEVIER, AMSTERDAM, NL, vol. 1185, 28 November 2007 (2007-11-28), pages 246-255, XP022368747 ISSN: 0006-8993

## Description

### Technical Field

The invention relates to a medicament for use in treating Alzheimer's disease comprising as active ingredients a carbostyril derivative of the general formula: 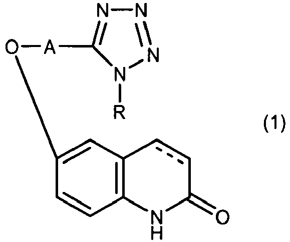 wherein A is a C₁₋₆ alkylene group, R is a cycloalkyl group, the bonding between 3- and 4-positions of the carbostyril skeleton is a single bond or a double bond, or a salt thereof; and donepezil or a salt thereof.

### Background Art

The carbostyril derivatives of formula (1) or salts thereof and the processes for the preparation thereof are disclosed in JP-63-20235-B and JP-55-35019-A. And it is known that the carbostyril derivatives (1) have platelet aggregation inhibition action, phosphodiesterase (PDE) inhibition action, antiulcer, hypotensive action and antiphlogistic action, and are useful as an antithrombotic agent, a drug for improving cerebral circulation, an antiinflammatory agent, an antiulcer drug, an antihypertensive drug, an antiasthmatic drug, a phosphodiesterase inhibitor, etc. In addition, it is known that the compounds are also useful as a medicament for treating allergic disease (JP-5-320050-A). It is also known that the carbostyril derivatives (1) are a medicament for treating Alzheimer's disease (JP-2006-518732-A).

The number of patients suffering from dementia in Japan was estimated at 1,890,000 people and the prevalence rate thereof was estimated at 7.6% in 2005, and thus the number and the rate have been increasing with the advance of aging of society. It is understood that the number of patients suffering from Alzheimer's disease (AD) occupies more than half of the total number of dementia patients.

The major part of AD is thought to be a sporadic case, but familial AD is thought to be included at about 10% which may develop with autosomal dominant inheritance due to a missense mutation of gene such as amyloid precursor protein gene, presenilin-1 and presenilin-2. Especially, women are more apt to suffer from AD. The most important risk factor of AD is an aging. The prevalence rate of AD increases with age, and thus the majority of AD is late-onset AD which develops on/after 65 years old. AD patients may show various pathologic conditions depending on the disorder of a neurotransmitter such as acetylcholine, the formation of senile plaques through the intracerebral accumulation of amyloid β protein, the intraneuronal accumulation of abnormal filaments comprising phosphorylated tau and other substances, the severe atrophy of a brain through shedding neuronal cells, etc. The core symptoms of AD include memory impairment, aphasia, cognitive impairment, disorder of executive function, and associated symptom; many of which are euphoric. However, some symptoms of AD exhibit adverse conditions such as lack of motivation, depression, bad temper, ill temper from the beginning of the onset.

For the core symptoms of AD, donepezil hydrochloride (commercial name: Aricept) is broadly used in clinical practice (JP-2578475-B). Further donepezil is known as useful therapeutic agent for the treatment of Alzheimer's disease (US 2004/229913). For the associated symptom such as insomnia, ill temper, and paranoia, donepezil hydrochloride is also useful as a palliative therapy (Japanese Journal of Psychiatric Treatment, Vol.21, Supplement, Page. 302-305, October 15, 2006, Tsuneyoshi Ohta, Heii Arai). However, the effect of the palliative therapy with donepezil hydrochloride is apt to descend as used in a long term. Thus, donepezil hydrochloride also has a problem that it is hard to continuously and sufficiently suppress the development of the pathologic condition since the medicament is necessary to be administered in a long-term.

### Disclosure of Invention

As mentioned above, donepezil hydrochloride (commercial name: Aricept^{®}) has been widely used as a medicament for treating Alzheimer's disease, however, it has been still desired to develop a more effective medicament for treating Alzheimer's disease which can suppress lowering of the therapeutic effect of donepezil hydrochloride through the long-term administration.

The present inventors have intensively studied a new medicament for treating Alzheimer's disease, and have found that a combination or a drug combination of a carbostyril derivative of the above formula (1), especially 6-[4-(1-cyclohexyl-1H-tetrazol-5-yl)butoxy]-3,4-dihydrocarbostyril (cilostazol) or a salt thereof, and donepezil or a salt thereof exhibits an excellent synergistic action for treating Alzheimer's disease, and then have accomplished the present invention. Especially, the present inventors have found that the combination of the present invention could exhibit an excellent effect improving the action of donepezil hydrochloride which had descended due to long-term administration of donepezil hydrochloride. In addition, the combination or the drug combination of the present invention exhibits fast-acting and low-toxicity, and hence it can be administered over long term. The present invention is also a useful medicament for treating Alzheimer's disease from the viewpoint of safety.

The present invention provides a medicament for use in treating Alzheimer's disease comprising a carbostyril derivative of the general formula: 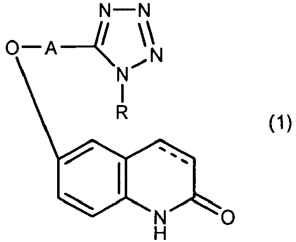 wherein A is a C₁₋₆ alkylene group, R is a cycloalkyl group, the bonding between 3- and 4-positions of the carbostyril skeleton is a single bond or a double bond, or a salt thereof, and donepezil or a salt thereof as active ingredients.

In a preferred embodiment the medicament for use in treating Alzheimer's disease comprises 6-[4-(1-cyclohexyl-1H-tetrazol-5-yl)butoxy]-3,4-dihydrocarbostyril (cilostazol) or a salt thereof, and donepezil or a salt thereof as active ingredients.

In a further preferred embodiment the medicament for use in treating Alzheimer's disease comprises the carbostyril derivative (1) and donepezil hydrochloride as active ingredients. The present invention also provides the use of the carbostyril derivative (1) or a salt thereof, and donepezil or a salt thereof for the preparation of a medicament for treating Alzheimer's disease.

According to the present invention, the combination of the carbostyril derivative (1), especially 6-[4-(1-cyclohexyl-1H-tetrazol-5-yl)butoxy]-3,4-dihydrocarbostyril or a salt thereof, and donepezil hydrochloride exhibits effective therapeutic and prophylactic action for Alzheimer's disease.

### Brief Description of Drawings

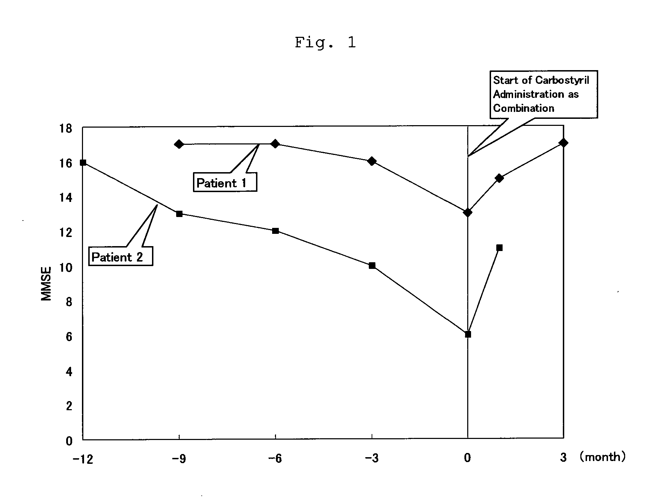

Fig. 1 denotes therapeutic effect for Alzheimer's disease using donepezil hydrochloride together with cilostazol as a combination.

### Best Mode for Carrying Out the Invention

The carbostyril derivative which is comprised in the medicament in combination with donepezil or a salt thereof is a tetrazolylalkoxy-dihydrocarbostyril derivative of the formula: 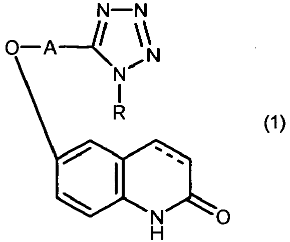 wherein A is a C₁₋₆ alkylene group, R is a cycloalkyl group, the bonding between 3- and 4-positions of the carbostyril skeleton is a single bond or a double bond, or a salt thereof.

In the above formula (1), the cycloalkyl group includes C₃-C₈ cycloalkyl groups such as cyclopropyl, cyclobutyl, cyclopentyl, cyclohexyl, cycloheptyl, and cyclooctyl. The preferred cycloalkyl group is cyclohexyl. The C₁₋₆ alkylene group includes methylene, ethylene, propylene, tetramethylene, butylene, and pentylene, among which the preferred one is tetramethylene.

The preferred carbostyril derivative is 6-[4-(1-cyclohexyl-1H-tetrazol-5-yl)butoxy]-3,4-dihydrocarbostyril, which has been put on the market in the trade name of cilostazol as an antiplatelet agent.

The carbostyril derivative (1) can be easily converted to a salt thereof by getting it treated with a pharmaceutically acceptable acid. The acid includes, for example, inorganic acids such as hydrochloric acid, sulfuric acid, phosphoric acid, and hydrobromic acid; and organic acids such as oxalic acid, maleic acid, fumaric acid, malic acid, tartaric acid, citric acid, and benzoic acid.

These carbostyril derivatives (1) and salts thereof and processes for preparation thereof are disclosed in JP-55-35019-A (relevant to U.S. Patent 4,277,479).

The other active ingredient is donepezil whose chemical name is 1-benzyl-4-[(5,6-dimethoxyindan-1-one)-2-yl]methylpiperazine. A hydrochloride thereof has been put on the market as a medicament for treating Alzheimer's disease (donepezil hydrochloride, commercial name: Aricept^{®}). This compound is disclosed in JP-2578475-B. 1-Benzyl-4-[(5,6-dimethoxyindan-1-one)-2-yl]methylpiperazine of the invention can be easily transformed to a salt form thereof using a pharmaceutical acceptable acid.

The acid includes, for example, inorganic acids such as hydrochloric acid, sulfuric acid, phosphoric acid and hydrobromic acid, and organic acids such as oxalic acid, maleic acid, fumaric acid, malic acid, tartaric acid, citric acid and benzoic acid. Amongst them, a hydrochloride thereof, donepezil hydrochloride is preferred.

These active ingredients, the carbostyril derivative (1) or a salt thereof and donepezil or a salt thereof may be administered together or separately, at the same time or different time. These ingredients may usually be used in a conventional pharmaceutical formulation. Then, these ingredients may be prepared in a single dosage form or in separate dosage forms.

The dose of these active ingredients is not limited to a specific range. The carbostyril derivatives (1) or a salt thereof may be used in an amount of 50 to 200 mg/day per an adult (50 kg of body weight), which is administered once a day or two to several times per day. Donepezil may be used in an amount of about 0.1 to 300 mg/day, preferably about 1 to 10 mg/day per an adult (50 kg of body weight), which is usually administered one to four times per day. When these ingredients are prepared in a single dosage form, they are incorporated in a ratio of 0.025 to 1.0 parts by weight of donepezil per 1 part by weight of the carbostyril derivative (1) or a salt thereof. And, the drug combination may include the sum of the ingredients in 0.1 - 70 % (w/w) per the preparation, but not limited thereto. The dosage form used for the medicament of the present invention includes, for example, the dosage forms exemplified in JP-10-175864-A, and typically an oral solid dosage form such as tablets and capsules, an oral liquid dosage form such as syrups and elixirs, a parenteral dosage form such as injections, and an inhalant.

The medicament of the invention such as tablets, capsules, liquid for oral administration may be prepared by a conventional method. The tablets may be prepared by mixing the active ingredient(s) with conventional pharmaceutical carriers such as gelatin, starches, lactose, magnesium stearate, talc and gum arabic. The capsules may be prepared by mixing the active ingredient(s) with inert pharmaceutical fillers or diluents and filling hard gelatin capsules or soft capsules with the mixture. The oral liquid preparations such as syrups or elixirs are prepared by mixing the active ingredient(s) with sweetening agents (e.g. sucrose), preservatives (e.g. methylparaben, propylparaben), colorants and flavors. The medicament for parenteral administration may also be prepared by a conventional method, for example, by dissolving the active ingredient(s) of the medicament in a sterilized aqueous carrier, preferably water or a saline solution. The preferred liquid medicament suitable for parenteral administration is prepared by dissolving the daily dose of the active ingredients as mentioned above in water and an organic solvent and further in a polyethylene glycol having a molecular weight of 300 to 5000, in which preferably a lubricant such as sodium carboxymethylcellulose, methylcellulose, polyvinylpyrrolidone, and polyvinyl alcohol is incorporated. Preferably, the above liquid preparations may further comprise a disinfectant (e.g. benzyl alcohol, phenol, thimerosal), a fungicide, and further optionally an isotonic agent (e.g. sucrose, sodium chloride), a topical anesthetic, a stabilizer and a buffer. In view of keeping stability, the medicament for parenteral administration may be put in capsules, followed by removing the aqueous medium by a conventional lyophilizing technique. The medicament can be recovered into a liquid medicament by dissolving in an aqueous medium when used. The inhalants may be prepared by a conventional method. That is, the inhalants may be prepared by getting an active compound to a powder or liquid state, mixing it into propellants and/or carriers for inhalant, and charging an appropriate vaporizer with the mixture. Ordinarily, a mechanical powder vaporizer can be used when the active compound is a powder, and a vaporizer such as a nebulizer can be used when the compound is a liquid. In addition, the inhalant may optionally comprise a surfactant, an oil, a flavor, a cyclodextrin or a derivative thereof which has been used when necessary.

The examples of the above-mentioned additive agents, processes thereof, or other things include what JP-10-175864-A discloses.

### Example

To two women suffering from Alzheimer's disease (one was 63 years old and the other was 52 years old), donepezil hydrochloride (Aricept^{®}) had been administered in a dose of 5 mg/day for 12 months and 9 months, respectively. During under the administration, the mini-mental state examination (MMSE) was carried out for the women (Journal of psychiatric research. 1975 Nov; 12 (3): 189-98.), and the result was shown in Table 1 and Figure 1. At the beginning time of the experiment (0 month), the administration of cilostazol in a dose of 100 mg/day started as the combination with donepezil hydrochloride. At that time, the MMSE score which had been descending until that time was quickly enhanced. According to the above result, it has found that the administration of cilostazol as the combination with donepezil hydrochloride can recover the effect of donepezil hydrochloride which tended to descend due to long-term administration of donepezil hydrochloride. And it has also found that the combination of donepezil hydrochloride and cilostazol can provide a potent therapeutic effect for dementia such as Alzheimer's disease.

**Table 1**

| Patient No. | Age | Sex | MMSE Score | | | | | | |
|---|---|---|---|---|---|---|---|---|---|
| | | | -12 Ms | -9 Ms | -6 Ms | -3 Ms | 0 M | 1 M | 3 Ms |
| 1 | 63 | F | - | 17 | 17 | 16 | 13 | 15 | 17 |
| 2 | 52 | F | 16 | 13 | 12 | 10 | 6 | 11 | - |

| | | | | | | | | | |
|---|---|---|---|---|---|---|---|---|---|
| M(s) denotes "month(s)". The administration of cilostazol in the combination started at 0 M. | | | | | | | | | |

## Claims

1. A medicament for use in treating Alzheimer's disease comprising as active ingredients a carbostyril derivative of the general formula: 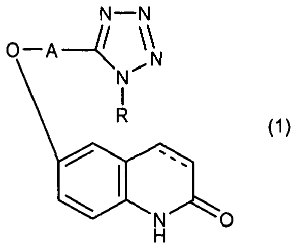 wherein A is a C₁₋₆ alkylene group, R is a cycloalkyl group, the bonding between 3- and 4-positions of the carbostyril skeleton is a single bond or a double bond, or a salt thereof; and donepezil or a salt thereof.

2. The medicament for use in treating Alzheimer's disease of claim 1, wherein the carbostyril derivative is 6-[4-(1-cyclohexyl-1H-tetrazol-5-yl)butoxy]-3,4-dihydrocarbostyril or a salt thereof.

3. The medicament for use in treating Alzheimer's disease of claim 1 or 2, wherein the salt of donepezil is donepezil hydrochloride.

4. Use of the carbostyril derivative or a salt thereof as set forth in claim 1 or 2, and donepezil or a salt thereof for the preparation of a medicament for treating Alzheimer's disease.

5. The use of claim 4, wherein the salt of donepezil is donepezil hydrochloride.

## Patentansprüche

1. Medikament zur Verwendung bei der Behandlung der Alzheimer-Krankheit, umfassend als Wirkstoffe ein Carbostyrilderivat der allgemeinen Formel 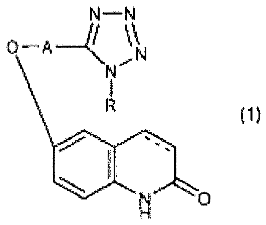 wobei A eine C₁₋₆-Alkylengruppe ist, R eine Cycloalkylgruppe ist, die Bindung zwischen den Positionen 3 und 4 des Carbostyrilgerüsts eine Einfachbindung oder eine Doppelbindung ist, oder ein Salz davon sowie Donepezil oder ein Salz davon.

2. Medikament zur Verwendung bei der Behandlung der Alzheimer-Krankheit gemäß Anspruch 1, wobei es sich bei dem Carbostyrilderivat um 6-[4-(1-Cyclohexyl-1H-tetrazol-5-yl)butoxy]-3,4-dihydrocarbostyril oder ein Salz davon handelt.

3. Medikament zur Verwendung bei der Behandlung der Alzheimer-Krankheit gemäß Anspruch 1 oder 2, wobei es sich bei dem Salz von Donepezil um Donepezil-Hydrochlorid handelt.

4. Verwendung des Carbostyrilderivats oder seines Salzes gemäß Anspruch 1 oder 2 und von Donepezil oder seines Salzes für die Herstellung eines Medikaments zur Behandlung der Alzheimer-Krankheit.

5. Verwendung gemäß Anspruch 4, wobei es sich bei dem Salz von Donepezil um Donepezil-Hydrochlorid handelt.

## Revendications

1. Médicament pour une utilisation dans le traitement de la maladie d'Alzheimer comprenant comme ingrédients actifs un dérivé de carbostyrile de la formule générale : 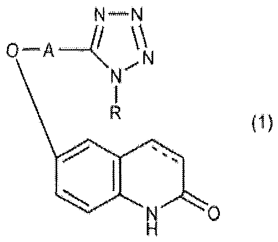 dans laquelle A est un groupe alkylène en C₁₋₆, R est un groupe cyclo-alkyle, la liaison entre les positions 3 et 4 du squelette carbostyrile est une liaison simple ou une liaison double,
ou un sel de celui-ci ; et du donépézil ou un sel de celui-ci.

2. Médicament pour une utilisation dans le traitement de la maladie d'Alzheimer selon la revendication 1, dans lequel le dérivé de carbostyrile est le 6-[4-(1-cyclohexyl-1H-tétrazol-5-yl)butoxy]-3,4-dihydrocarbostyrile ou un sel de celui-ci.

3. Médicament pour une utilisation dans le traitement de la maladie d'Alzheimer selon la revendication 1 ou 2, dans lequel le sel de donépézil est l'hydrochlorure de donépézil.

4. Utilisation du dérivé de carbostyrile ou d'un sel de celui-ci selon la revendication 1 ou 2, et de donépézil ou d'un sel de celui-ci pour la préparation d'un médicament pour le traitement de la maladie d'Alzheimer.

5. Utilisation selon la revendication 4, dans laquelle le sel de donépézil est l'hydrochlorure de donépézil.
